# EUROPEAN PATENT SPECIFICATION

(11) **EP 1 027 373 B1**
(45) Date of publication and mention of the grant of the patent: **06.09.2006**
(21) Application number: 98956204.6
(22) Date of filing: 30.10.1998
(51) Int. Cl.: C07K 14/47, A61K 38/17

(54) **MUC-1 DERIVATIVES AND THEIR USE IN TREATING CANCER-ASSOCIATED MUC-1 MUCIN-INDUCED IMMUNOSUPPRESSION**
MUC-1 DERIVATE UND DEREN VERWENDUNG ZUR BEHANDLUNG VON KREBSASSOZIIERTER MUC-1 MUCIN INDUZIERTER IMMUNOSUPPRESSION
DERIVES DE MUC-1 ET LEUR UTILISATION POUR TRAITER L'IMMUNODEPRESSION INDUITE PAR LA MUCINE MUC-1 ET ASSOCIEE AU CANCER

(30) Priority: 31.10.1997 US 64146; 12.11.1997 US 65209
(43) Date of publication of application: 16.08.2000
(60) Divisional application: 03077651.2
(73) Proprietor: BIOMIRA, INC., Edmonton, Alberta T6N 1H1 (CA)
(72) Inventor: AGRAWAL, Babita, Edmonton, Alberta T6H 4M5 (CA); REDDISH, Mark, Austin, Edmonton, Alberta T6H 3S7 (CA); LONGENECKER, Bryan, Michael, Edmonton, Alberta T6R 1C8 (CA)
(74) Representative: Maschio, Antonio
(86) International application number: PCT/US1998/022644
(87) International publication number: WO 1999/023114

(56) References cited:
- WO-A-90/05142
- WO-A-96/22067
- AGRAWAL B ET AL: "In vitro induction of MUC-1 peptide-specific type 1 T lymphocyte and cytotoxic T lymphocyte responses from healthy multiparous donors." JOURNAL OF IMMUNOLOGY, (1996 SEP 1) 157 (5) 2089-95. JOURNAL CODE: IFB. ISSN: 0022-1767., XP002098870 United States
- COVENTRY B J ET AL: "Lack of IL-2 cytokine expression despite Il-2 messenger RNA transcription in tumor-infiltrating lymphocytes in primary human breast carcinoma: selective expression of early activation markers." JOURNAL OF IMMUNOLOGY, (1996 MAY 1) 156 (9) 3486-92. JOURNAL CODE: IFB. ISSN: 0022-1767., XP002098871 United States
- AGRAWAL E.A.: "Cancer-associated MUC1 mucin inhibits T-cell proliferation, which is reversible by IL-2" NATURE MEDICINE, vol. 4, no. 1, January 1998, pages 43-49, XP002098872

## Description

### Background of the Invention

MUC-1 mucin is a high molecular weight glycoprotein with a protein core consisting of tandem repeats of a 20 amino acid sequence and highly-branched carbohydrate side chains, Many human adenocarcinomas, such as breast, colon, lung, ovarian and pancreatic cancers, abundantly over express and secrete under-glycosylated MUC-1 protein. Importantly, a high level of MUC-1 mucin expression is associated with high metastatic potential and poor prognosis. MUC-1 is, therefore, a clinically significant marker for these cancers.

High serum MUC-1 levels in cancer patients also have been correlated with immunosuppression in metastatic adenocarcinoma patients who received active specific immunotherapy. The data herein show that MUC-1 is, at least in part, directly responsible for this immunosuppression.

Cytokines, such as IL-2, have been used clinically to support immunotherapy of various cancers. The use of cytokines, although capable of reversing MUC-1-induced immunosuppression, leads to a relatively non-specific activation of a wide variety of immune cells.

A need exists, therefore, for improved immunotherapeutic medicaments, and regimens using them, that reduce or eliminate MUC-1-induced suppression and/or anergy of immune responses.

### Summary of the Invention

It is therefore an object of the invention to provide improved immunotherapeutic medicaments that are useful in relieving anergy and/or suppression of the immune system. According to this object, novel medicaments are provided which are active in relieving immune cell anergy and/or immunosuppression. One class of such medicaments comprises MUC-1 derivatives which reverse MUC-1-mediated immunosuppression. In one embodiment, MUC-1 derivatives are provided which comprise a peptide derived from the MUC-1 core sequence PDTRPAPGSTAPPAHGVTSA, and permutations thereof. In another embodiment, MUC-1 derivatives are provided which comprise a MUC-1 core peptide derivative fused to a stimulatory antigen. In yet another embodiment, MUC-1 derivatives are provided which comprise a MUC-1 core peptide derivative fused to a cytokine.

It is another object of the invention to provide pharmaceutical compositions suitable for therapeutic applications requiring reversal of immune cell anergy and/or immunosuppression. According to this object, pharmaceutical compositions are provided which comprise MUC-1 derivatives admixed with a pharmaceutically acceptable excipient.

It is yet another object of the invention to provide methods of treatment which relieve antigen-induced immunosuppression and/or immune cell anergy. According to this object, methods are provided which comprise administering to a patient in need of treatment a MUC-1 derivative.

### Brief Description of the Drawings

Figure 1 depicts the ability of MUC-1 to suppress the immune response to the various stimuli indicated.
Figure 2, panel (a) shows a similar suppression by larger tandem repeats of the MUC-1 core sequence, but not the single repeat 16-mer. Panels (b) and (c) show reversal of MUC-1 suppression by Anti-CD28 and IL-2.
Figure 3, depicts alleviation of MUC-1-induced anergy/suppression by 16-mer peptide BP₁₆, derived from the MUC-1 core sequence. The left panel is the medium control and the right panel is the experimental, demonstrating specific anergy/suppression alleviation.

### Detailed Description

Mucins are a family of large glycoproteins of greater than 200 kDa molecular weight. Some mucins, such as MUC-1, are membrane-bound molecules with an extended extracellular domain composed of tandem repeats of amino acid (aa) sequences which contain numerous potential O-glycosylation sites. Devine, *et al. BioEssays* 14: 619 (1992).

Numerous clinical studies have suggested that mucinous tumor antigens, both expressed on the cell surface of tumor cells and shed from the surface of tumor cells, are associated with a poor prognosis of a variety of cancer types. See, for example Kobayashi *et al. J. Clinical Oncol,* 10: 95-101 (1992).

In a recent study, we demonstrated that cancer patient- derived MUC-1 mucin produces inhibition of specific human T cell responses. Agrawal et al. Nature Medicine, 4:43-49 (1998). In addition, MUC-1 mucin-derived long synthetic peptides, but not small peptides, produce the same T cell suppression. These MUC-1-derived peptides comprised multiple tandem repeats of the specific 20 amino acid core repeat of MUC-1, indicating the importance of the repeats in this physiological effect Surprisingly, however, when a peptide which was smaller than three multiples of the 20 amino acid core repeat were tested, the inventors found that it did not induce anergy.

The portion of MUC-1 believed responsible for its specific immunosuppressive properties, therefore, is composed of multiple tandem repeats of the twenty amino acid sequence. The inventors hypothesize that multiple repeats are needed to induce immunosuppression because simultaneous interaction with multiple cell surface receptors is required. Thus, cross-linking of multiple receptors, and possibly capping of the crosslinked receptors, may be required for immunosuppression. Accordingly, any medicament that can specifically disrupt this process may be useful in reversing or even preventing MUC-1-induced immunosuppression.

The present invention contemplates MUC-1 derivatives, including specific peptides and peptide mimetics which, as demonstrated by assays, such as those set forth below, have the ability to reverse or prevent MUC-1-induced anergy/immunosuppression. Such compounds have the ability specifically to interfere with the adverse, pathological activities of MUC-1. As used herein, the terms "anergy" and "immunosuppression" are used interchangeably and specifically incorporate all attributes ascribed to these terms, individually and collectively, by the immunological arts.

In view of the foregoing, one class of useful compound will be that which disrupts the binding of MUC-1 to a cell surface receptor. This disruption can occur by competitively inhibiting the binding of MUC-1. Thus, in a prophylactic application, the compound would occupy the site through which MUC-1 mediates its immunosuppressive effects, thereby preventing MUC-1 binding altogether. In another application, the inventive compounds may be used to reverse MUC-1 binding by displacing it from the receptor.

### Compounds of the Invention

The inventive compounds are herein generically termed "MUC-1 derivatives." The compounds are not limited, however, to those specifically derived from MUC-1, but include the entire class of compounds which exhibit activity in relieving MUC-1-induced immunosuppression.

An important class of MUC-1 derivatives includes peptide derivatives. Specific peptide-based derivatives include those derived from the sequence of the core repeat of native MUC-1. In one embodiment, the peptide would include the extracellular tandem repeat region of MUC-1, which includes repeats of the amino acid sequence DTRP (Asp-Thr-Arg-Pro).

Preferably these tandem repeats include the sequence SAPDTRP (Ser-Ala-Pro-Asp-Thr-Arg-Pro).

A MUC-1 "core repeat," "core sequence" or MUC-1 core" as used herein generally refers to that present in the native MUC-1 molecule, the sequence of which is well known to the artisan, which comprises the 20 amino acid sequence PDTRPAPGSTAPPAHGVTSA (Pro-Asp-Arg-Thr-Pro-Ala-Pro-Gly-Ser-Thr-Ala-Pro-Pro-Ala-His-Gly-Val-Thr-Ser-Ala).

Deletion derivatives, including truncations and internal deletions, are especially useful. One particularly useful MUC-1 derivative of this class is a 16 amino acid peptide of the sequence GVTSAPDTRPAPGSTA.

Some preferred peptide-based MUC-1 derivatives comprise one, or less than one, peptide core repeat of the MUC-1 mucin. Of course, a minimum size of at least a dipeptide is inherent in such derivatives, since they contain peptide bonds. Thus, a recitation of "at most one MUC-1 core repeat" contemplates a minimum dipeptide. This, of course, is subject to such a molecule having the requisite anergy/immunosuppression alleviating properties. Thus, typical MUC-1 core repeats will have a minimum size of at least about 5 amino acids, for example SAPDTRP, with a class of especially useful repeats having a minimum size of about 10 amino acids. The maximum size of "at most one MUC-1 core repeat" would be 20 amino acids, as prescribed by the native length.

Other useful MUC-1 derivatives include glycosylated or non-glycosylated peptides. Glycosylation may improve circulating half-life and allow modulation of the immunosuppression-reversing characteristics of MUC-1 derivatives. Glycosylation can be biological or non-biological. For example, biologically relevant N- or O-linked carbohydrates are envisioned. Altentatively, other derivatives, such a succinate, may be employed. Other chemical modifications, such as with polyethylene glycols are also contemplated.

Although small peptides may be preferable from both economic and certain technical perspectives, larger molecules are also contemplated. Thus, peptide-based MUC-1 derivatives may be combined with other useful therapeutic agents, yielding enhanced properties. They may be so combined, for example, covalently or electrostatically. Ideally these other therapeutic agents will be immunomodulators, and preferably will have immunostimulatory properties. Although non-protein agents are contemplated, the additional therapeutic agents are preferably proteins, which generically include peptides. Some particularly useful protein therapeutics include cytokines.

In one example, fusion proteins comprise an inventive peptide fused to a cytokine. Such fusions are expected to have hybrid properties of reversing MUC-1-induced immunosuppression and more broadly inducing the immune response. Moreover, due to the interaction of the MUC-1-based peptide component with suppressed T cells, the cytokine will be in a close physical proximity with the target cell, which may allow a specific cytokine-mediated induction of the very cells being de-repressed by the peptide portion of the MUC-1 derivative. Not only will immunosuppression be relieved, specific immunostimulation of the same T cell population will be achieved.

Particularly useful cytokines include those with immunostimulatory activity. Some preferred cytokines include the interleukins (ILs), and especially IL-2. Other useful cytokines include, for example, IL-1, IL-4, IL-7, IL-10, IL-12, and γ-interferon. MUC-1 may be linked to these molecules with the aid of recombinant DNA techniques. Altematively the proteins may be attached to each other using known multivalent cross-linking agents. Both of those techniques or well known to the artisan and may be found in any standard compilation of laboratory methods, such as the current versions of Sambrook *et al.,* 1989, MOLECULAR CLONING. A LABORATORY MANUAL, Cold Spring Harbor Press, N.Y.; and Ausubel *et al.,* 1989, CURRENT PROTOCOLS IN MOLECULAR BIOLOGY, Green Publishing Associates and Wiley Interscience, N.Y.

Specific useful MUC-1 derivatives can be derived from purified MUC-1, or portions thereof, produced by native sources or recombinant DNA methodology, by methods that include digestion with enzymes such as pepsin or papain. Alternatively, peptides encompassed by the present invention can be synthesized using an automated peptide synthesizer such as those supplied commercially by Applied Biosystems, Multiple Peptide Systems and others, or they may be produced manually, using techniques well known in the art See Geysen *et al., J. Immunol. Methods* 102: 259 (1978). Glycosylated and other forms of peptide or protein MUC-1 derivatives may be made according to methods well known in the art.

Although most preferred MUC-1 derivatives are protein- (or peptide-) based, other derivatives are contemplated For example, small molecules which are amino acid or peptide mimetics may be useful. Rational design of such molecules is possible using methods known in the art. Using, for example, space-filling models, otherwise structurally unrelated compounds may be made to mimic protein-based MUC-1 derivatives. The usefulness of these MUC-1 derivatives can be confirmed using routine assays, such as those presented in the examples.

### Pharmaceutical Compositions of the Invention

The inventive compositions may be formulated for administration a variety of ways. The pharmaceutical compositions of the invention generally contain a pharmaceutically effective amount of an inventive compound. Preferably, the compound is admixed with a pharmaceutically effective vehicle (excipient).

A suitable formulation will depend on the nature of the specific medicament chosen, whether the treatment is *in vivo* or *ex vivo,* the route of administration desired and the judgment of the attending physician. Suitable formulations and pharmaceutically effective vehicles, can be found, for example, in REMINGTON'S PHARMACEUTICAL SCIENCES, chapters 83-92, pages 1519-1714 (Mack Publishing Company 1990) (Remington's).

Preferred vehicles include liposomes. See, for example, Remington's at 1691-92. Thus, the inventive compositions may also be formulated, and administered, in combination with other known medicaments, which may provide complementary anergy/immunosuppression relieving activity, in liposomal formulations. Preferred other medicaments include immunomodulators, such as the cytokines discussed above.

The pharmaceutical compositions of the invention also may be formulated with stimulatory antigens, such as adjuvants. Such adjuvants are well known in the vaccine arts and typically function to enhance the immune response. Thus, preferred adjuvants useful in the invention are characterized by enhancing the ability of the inventive medicaments described herein to relieve antigen-induced immunosuppression/anergy. Some examples of well-known and useful adjuvants include those derived from bacterial lipopolysaccharides, such as lipid A, monophosphoryl lipid A.

### Methods of the Invention

The inventive methods typically involve administering to a patient in need of treatment, an effective amount of at least one MUC-1 derivative, as described above. Of course, administration of the above pharmaceutical compositions is fully interchangeable with administration of any MUC-1 derivative in all of the inventive methods. Other methods contemplate combination therapy with at least one MUC-1 derivative, in conjunction with at least one other medicament. The patient may be a human or non-human animal. A patient typically will be in need of treatment when suffering from anergy/immunosuppression, which may be induced by MUC-1.

Although primary applicability will be to MUC-1-induced disorders, it is contemplated that the inventive methods may apply more generally. Thus, the biological activity observed herein may also have aspects which are not simply antigen-specific, but are also relevant to reversing anergy/immunosuppression in general. Such a situation typically will arise due to antigenic cross-reactivity. Thus, other anergy- or immunosuppression-inducing antigens may contain the same or overlapping epitopes as MUC-1. Accordingly, the compounds disclosed herein will be applicable in treating such disorders.

The inventive methods may be employed *in vivo* or *ex vivo.* In a typical *ex vivo* method, for example, peripheral T cells may be isolated from patients, treated with at least one MUC-1 derivative, alone or in combination, and re-infused into the patient..

Administration during *in vivo* treatment may be by any number of routes, including parenteral and oral. Specific preferred routes include direct injection into the tumor or the draining lymph nodes. Thus, for example, the tumor infiltrating lymphocytes within the tumor, which are known to be immunosappressed, will be specifically targeted and de-repressed.

MUC-1 derivatives may be administered alone, in combination with each other, or in combination with other medicaments. Ideally these other medicament agents will be immunomodulators, and preferably will have immunostimulatory properties. Both protein and non-protein agents are contemplated. Some particularly useful protein-based agents include stimulatory antigens and cytokines, as provided above. For example, cytokines may be coadministered, simultaneously or in succession, with MUC-1 derivatives. Of course, MUC-1 derivatives also may be used in combination with other anti-neoplastic regimens.

The term "treating" in its various grammatical forms in relation to the present invention refers to preventing, curing, reversing, attenuating, alleviating, minimizing, suppressing or halting the deleterious effects of a disease state, disease progression, disease causative agent or other abnormal condition. Methods of prophylaxis are specifically encompassed by the term ''treatment."

Determining a pharmaceutically effective amount of MUC-1 derivative is well within the purview of the skilled clinician, and largely will depend on the exact identity of the inventive compound, particular patient characteristics, route of administration and the nature of the disorder being treated. General guidance can be found, for example, in the publications of the International Conference on Harmonisation and in REMINGTON'S PHARMACEUTICAL SCIENCES, chapters 27 and 28, pp. 484-528 (Mack Publishing Company 1990).

Determining a pharmaceutically effective amount specifically will depend on such factors as toxicity and efficacy of the medicament. Toxicity may be determined using methods well known in the art and found in the foregoing references. Efficacy may be determined utilizing the same guidance in conjunction with the methods described below in the Examples. A pharmaceutically effective amount, therefore, is an amount that is deemed by the clinician to be toxicologically tolerable, yet efficacious.

Efficacy, for example, is measured by alleviation or substantial alleviation of anergy or immunosuppression, in accord with the definition of "treating" discussed above. In quantitative terms, "substantial alleviation" will usually be at least a 50% effect, relative to a normal control, as measured by conventional immunoassays. Since it is usually desirable to achieve a greater degree of relief from immunosuppression/energy, a preferred effective amount provides a 75% reversal if immunosuppression/anergy. Most preferably; however, at least a 90% effect is obtained, which is considered essentially complete "alleviation."

The foregoing discussion and following examples are presented merely for illustrative purposes and are not meant to be limiting. Thus, one skilled in the art will readily recognize additional embodiments within the scope of the invention that are not specifically exemplified.

### EXAMPLES

### Example 1:

This example shows that adding purified human MUC-1 mucin to human T-cell cultures strongly inhibits T-cell proliferation against a strong allo-antigenic stimulus (or mitogenic stimulus) in vitro.

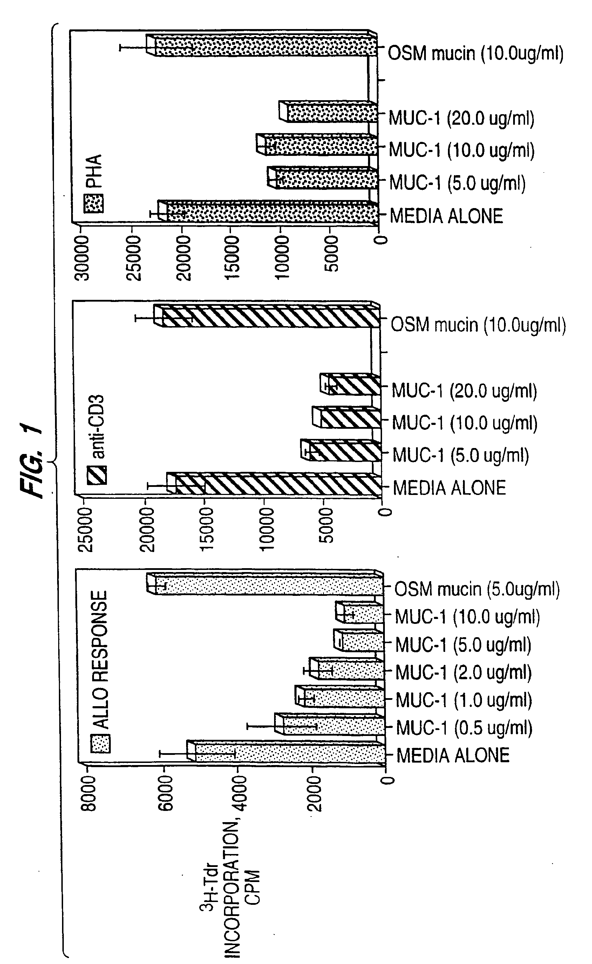

The mixed lymphocyte reaction is conducted by mixing the lymphocytes of HLA disparate individuals in in vitro tissue cultures. The "responder population" in this experiment is purified T-cells from one population, while the "stimulator" population in this experiment is the peripheral blood lymphocytes obtained from an HLA mismatched individual donor. The two cell populations were mixed and cultured either in the presence or absence of various doses of B27.29 affinity purified MUC-1 mucin that was purified from a pleural effusion fluid. The results of this experiment are depicted in Figure 1.

In the experiment depicted in Figure 1, 10° T-cells were cultured in AIM V medium in presence of 10^{e} allo-PBLs (mitomycin C treated) with or without the indicated concentration of affinity purified MUC1 or OSM for 6-7 days. At this time the T-cells were harvested and plated at 10⁵/well in 96 well flat bottom plates and the polyclonal stimuli allo-PBLs (10⁵/well), or anti-CD3 (1 µg/ml) or PHA (0.2 µg/ml), in presence or absence of MUC 1 or OSM for 4 days. Each group was set up in a replicate of five wells. ³H-thymidine (1 µCi/well) was added and the culture plates were further incubated for 18-24 h before harvesting. ³H-thymidine incorporation into the DNA of proliferating T-cells was measured by liquid scintillation counting. The data are shown as mean CPM of the replicate wells ± standard deviations. Each experiment was repeated 4 times and data from one representative experiment is shown.

### Example 2

This example demonstrates the ability of synthetic peptides, having multiple tandem repeats of the MUC-1 core, to inhibit T cell proliferation and the failure of an embodied MUC-1 derivative to so inhibit.

### Mucins:

MUC-1 was purified from ascites fluid obtained from ovarian cancer patients, 2M sodium acetate at·pH 5 was added to the ascites fluids and centrifuged for 30 minutes at 20k rpm. After filtration through a 0.45 micron cellulose acetate filter, the solution was mixed with B27.29 Mab (Reddish *et al.,* J. Tumor Marker Oncol. 7:19-27 (1992)) CNBr coupled to sepharose 4B overnight, followed by washing with 1M NaCl/PBS. The affinity bound MUC-1 mucin was eluted with 50 mM diethanolamine (Fisher purified) in 150 mM NaCl at pH 11. The eluant was neutralized with 2M sodium acetate at pH 5. The affinity purified material was dialyzed against PBS and then sterile filtered with Nalgene 0.2 micron cellulose acetate syringe filter. The affinity purified MUC-1 mucin was quantified by using Truquant BR RIA assay (Biomira Diagnostics Inc., Roxdale, ON, Canada). For the calculation of amount of MUC-1 mucin, the conversion formula 1 BR unit as approximately 50 ng of MUC-1 mucin, was used,

Synthetic MUC-1 derivatives contained 1, 3, 4, 5 or 6 tandem repeats of the MUC-1 core and were approximately 16, 60, 80, 100 and 120 amino acids in length. The 16-mer (BP₁₆). contained the sequence GVTSAPDTRPAPGSTA. The other derivatives contained tandem repeats of the sequence TAPPAHGvTSAPDTRPAPGS;

Ovine submaxillary mucin (OSM) was employed as a control

### T cell cultures:

Enriched T cell populations were purified from buffy coats obtained from normal red cross donors using nylon wool columns by previously reported procedures. See, *e.g.,* Agrawal *et al.,* J. Immunol. 157: 2089-95 (1996) and Agrawal *et al,* J. ImmunoL'157: 3229-34 (1996). For the allo MLR, mitomycin C treated allogeneic PBLs were co-cultured with purified T cells in the presence or absence of affinity purified MUC-1 mucin or control OSM. In most of the experiments, the T cells were cultured 6-7 days in AIM V medium in the absence or presence of MUC-1, MUC-1 derivative or OSM at the iridicated concentration. After this time, the T cells were harvested, washed and cultured as indicated.

### Proliferation assay:

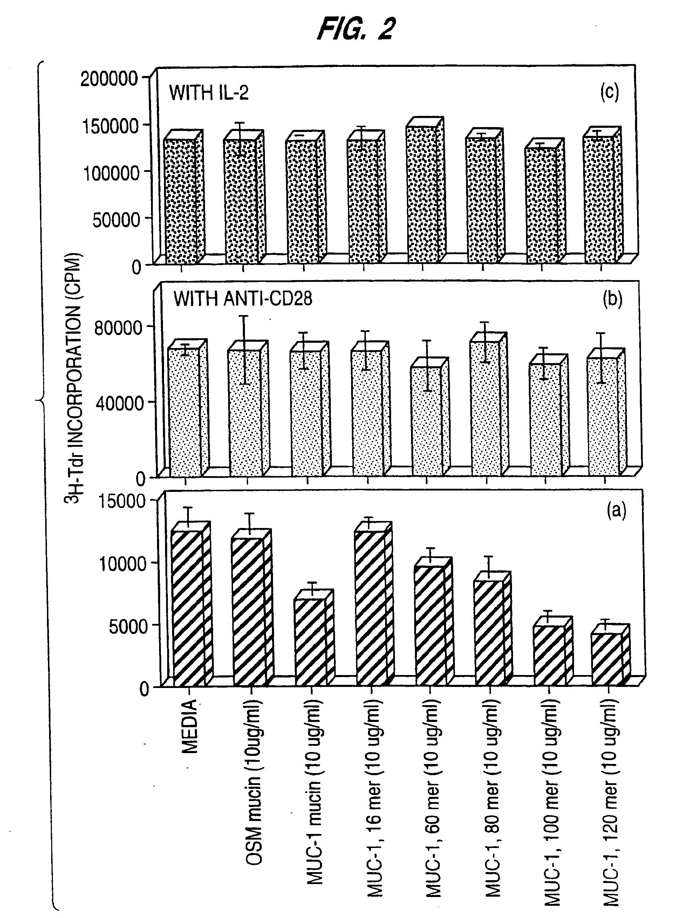

For the experiment depicted in Figure 2, purified T cells (10⁶/ml) were cultured in AIM V medium with allo PBLs in the absence or presence of MUC-1, MUC-1 derivative or OSM 10 µg/ml for 6-7 days. T cells were harvested and plated in 96 well flat bottom plates at 10⁵/well with allo PBLs (10⁵/well), in the presence or absence of affinity purified MUC-1, MUC-1 derivative or OSM. Control cultures were treated with either 50 U/ml IL-2 or 1 µg/ml anti-CD28 Mab. After 4 days of culture, ³H-thymidine (1 µCi/well) was added. The cells were harvested on the fifth day and ³H-thymidine incorporation was measured by liquid scintillation.

### Results:

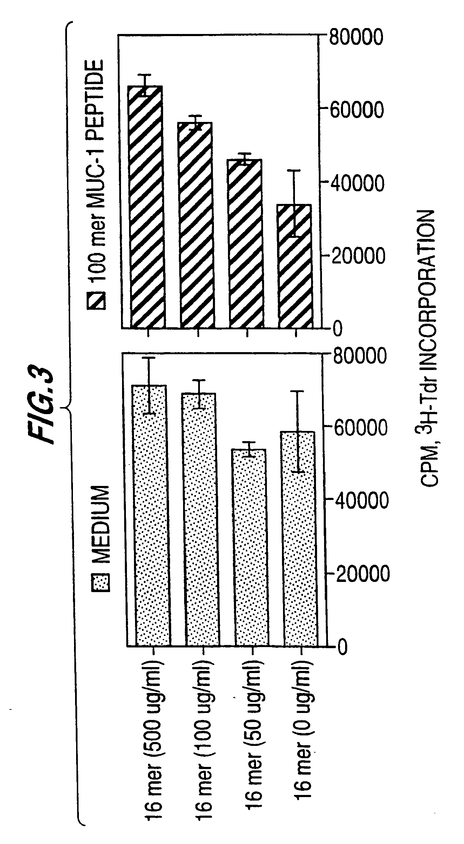

As seen in Figures 2 and 3, synthetic peptides containing 3-6 tandem repeats of the MUC-1 core significantly reduced the level ofT cell proliferation relative to control. This effect was not observed with a peptide containing a single repeat. Moreover, this effect was reversed by treatment with IL-2 or CD28 Mab. Table I demonstrates the statistical significance of these data as compared to the medium control.

**Table 1**

| Sample | p |
|---|---|
| 3 repeats | =0.0009 |
| 4 repeats | =0.0007 |
| 5 repeats | <0.0001 |
| 6 repeats | <0.0001 |

Table 2 demonstrates the statistical significance of these data compared to the OSM control.

**Table 2**

| Sample | p |
|---|---|
| 3 repeats | =0.036 |
| 4 repeats | =0.003 |
| 5 repeats | <0.0001 |
| 6 repeats | <0.0001 |

### Example 3

This example demonstrates the ability of a representative MUC-1 derivative to relieve MUC-1-induced immunosuppression As depicted in Figure 3, treatment with MUC-1 derivative BP₁₆ reverses suppression/anergy induced by a MUC-1 100-merpeptide).

In the experiment of Figure 3, purified T-cells (10⁶/ml) were cultured in AIM V medium with allo-PBLs in the presence (100 mer MUC1 peptide group; right panel) or absence (media group; left panel) of 100 mer MUC1 synthetic peptide (25 µg/ml) for 6-7 days. At this time, the T-cells were harvested and plated in 96 well flat bottom plates at 10⁵/well with allo-PBLs (10⁵/well) in the presence or absence of 100 mer MUC1 peptide (25 µg/ml), and the 16 mer MUC1 peptide (less than one tandem repeat) at varying doses. The wells were pulsed with ³H-thymidine (1 µCi/well) on the fourth day of culture followed by harvesting on the fifth day. ³H-Thymidine incorporation into the DNA of proliferating T-cells was measured by liquid scintillation counter. Data are shown as mean CPM ± standard deviations. Bach group was set up in replicates of 3 wells.

The foregoing discussion and following examples are presented merely for illustrative purposes and are not meant to be limiting. Thus, one skilled in the an will readily recognize additional embodiments within the scope of the invention that are not specifically exemplified.

## Claims

1. Use of a MUC-1 peptide in the manufacture of a medicament for relieving MUC-1 induced immunosuppression or anergy, wherein said MUC-1 peptide consists of the amino acid sequence GVTSAPDTRPAPGSTA.

2. Use according to claim 1, wherein said MUC-1 peptide is linked to a cytokine.

3. Use according to claim 2, wherein said cytokine is selected from the group consisting of IL-1, IL-4, IL-7, IL-10, IL-12, and γ-interferon.

4. Use according to claim 3, wherein said cytokine is IL-2.

5. Use according to claim 1, wherein said medicament further comprises a cytokine.

6. Use according to claim 5, wherein said cytokine is selected from the group consisting of IL-1, IL-4, IL-7, IL-10, IL-12, and γ-interferon.

7. Use according to claim 6, wherein said cytokine is IL-2.

8. Use according to claim 1, wherein said medicament further comprises a stimulatory antigen.

9. Use according to claim 8, wherein said stimulatory antigen is an adjuvant.

10. Use according to claim 9, wherein said adjuvant is derived from a bacterial lipopolysaccharide.

11. Use according to claim 10, wherein said bacterial lipopolysaccharide is lipid A or monophosphoryl lipid A.

12. Use according to any one of the preceding claims, wherein said MUC-1 peptide is glycosylated.

13. Use according to any one of the preceding claims, wherein an L-amino acid in the peptide is replaced with a D-amino acid.

14. Use of claim 1, wherein the medicament is admixed with a pharmaceutically effective vehicle.

15. Use of claim 14, wherein the pharmaceutically effective vehicle is a liposome.

## Patentansprüche

1. Verwendung eines MUC-1-Peptids bei der Herstellung eines Medikaments zum Lindern von MUC-1-induzierter Immunsuppression oder Anergie, wobei das MUC-1-Peptid aus der Aminosäuresequenz GVTSAPDTRPAPGSTA besteht.

2. Verwendung nach Anspruch 1, wobei das MUC-1-Peptid mit einem Zytokin verbunden ist.

3. Verwendung nach Anspruch 2, wobei das Zytokin aus der Gruppe ausgewählt ist, bestehend aus IL-1, IL-4, IL-7, IL-10, IL-12 und y-Interferon.

4. Verwendung nach Anspruch 3, wobei das Zytokin IL-2 ist.

5. Verwendung nach Anspruch 1, wobei das Medikament weiterhin ein Zytokin umfaßt.

6. Verwendung nach Anspruch 5, wobei das Zytokin aus der Gruppe ausgewählt ist, bestehend aus IL-1, IL-4, IL-7, IL-10, IL-12 und γ-Interferon.

7. Verwendung nach Anspruch 6, wobei das Zytokin IL-2 ist.

8. Verwendung nach Anspruch 1, wobei das Medikament weiterhin ein stimulatorisches Antigen umfaßt.

9. Verwendung nach Anspruch 8, wobei das stimulatorische Antigen ein Adjuvans ist.

10. Verwendung nach Anspruch 9, wobei das Adjuvans von einem bakteriellen Lipopolysaccharid abgeleitet ist.

11. Verwendung nach Anspruch 10, wobei das bakterielle Lipopolysaccharid Lipid A oder Monophosphoryl-Lipid A ist.

12. Verwendung nach einem der vorangegangenen Ansprüche, wobei das MUC-1-Peptid glycosyliert ist.

13. Verwendung nach einem der vorangegangenen Ansprüche, wobei eine L-Aminosäure in dem Peptid durch eine D-Aminosäure ersetzt ist.

14. Verwendung nach Anspruch 1, wobei das Medikament mit einem pharmazeutisch wirksamen Vehikel vermischt ist.

15. Verwendung nach Anspruch 14, wobei das pharmazeutisch wirksame Vehikel ein Liposom ist.

## Revendications

1. Utilisation d'un peptide MUC-1 dans la préparation d'un médicament destiné à soulager l'immunosuppression ou l'énergie induite par MUC-1, dans laquelle ledit peptide MUC-1 consiste en la séquence d'aminoacides GVTSAPDTRPAPGSTA.

2. Utilisation suivant la revendication 1, dans laquelle ledit peptide MUC-1 est lié à une cytokine.

3. Utilisation suivant la revendication 2, dans laquelle ladite cytokine est choisie dans le groupe consistant en IL-1, IL-4, IL-7, IL-10, IL-12 et interféron γ.

4. Utilisation suivant la revendication 3, dans laquelle ladite cytokine est la IL-2.

5. Utilisation suivant la revendication 1, dans laquelle ledit médicament comprend en outre une cytokine.

6. Utilisation suivant la revendication 5, dans laquelle ladite cytokine est choisie dans le groupe consistant en IL-1, IL-4, IL-7, IL-10, IL-12 et interféron γ.

7. Utilisation suivant la revendication 6, dans laquelle ladite cytokine est la IL-2.

8. Utilisation suivant la revendication 1, dans laquelle ledit médicament comprend en outre un antigène stimulateur.

9. Utilisation suivant la revendication 8, dans laquelle ledit antigène stimulateur est un adjuvant.

10. Utilisation suivant la revendication 9, dans laquelle ledit adjuvant est dérivé d'un lipopolysaccharide bactérien.

11. Utilisation suivant la revendication 10, dans laquelle ledit lipopolysaccharide bactérien est le lipide A ou le monophosphoryl-lipide A.

12. Utilisation suivant l'une quelconque des revendications précédentes, dans laquelle ledit peptide MUC-1 est glycosylé.

13. Utilisation suivant l'une quelconque des revendications précédentes, dans laquelle un L-aminoacide dans le peptide est remplacé par un D-aminoacide.

14. Utilisation suivant la revendication 1, dans laquelle le médicament est mélangé à un véhicule pharmaceutiquement efficace.

15. Utilisation suivant la revendication 14, dans laquelle le véhicule pharmaceutiquement efficace est un liposome.
